# EUROPEAN PATENT APPLICATION

(11) **EP 1 997 898 A1**
(43) Date of publication of application: **03.12.2008**
(21) Application number: 07109450.2
(22) Date of filing: 01.06.2007
(51) Int. Cl.: C12N 15/64, C12N 15/74

(54) **Means and methods for cloning nucleic acid sequences**

(71) Applicant: Rijksuniversiteit Groningen, 9712 CP Groningen (NL)
(72) Inventor: Geertsma, Eric Robin, 9727 JR Groningen (NL); Poolman, Berend, 9751 AC Haren (NL)
(74) Representative: van Loon, C.J.J.

(57) **Abstract**

The invention provides means and methods for efficiently cloning nucleic acid sequences of interest in micro-organisms that are less amenable to conventional nucleic acid manipulations, as compared to, for instance, *E.coli.* The present invention enables high-throughput cloning (and, preferably, expressing) of a nucleic acid of interest in a first kind of micro-organism, while an initial (preferably high throughput) cloning of the nucleic acid of interest is done in a second kind of micro-organism.

SEQUENCE LISTING

<110>  Rijksuniversiteit Groningen

<120>  Means and methods for cloning nucleic acid sequences

<130>  P81381EP00

<140>  EP 07109450.2
<141>  2007-06-01

<160>  41

<170>  PatentIn version 3.3

<210>  1
<211>  13
<212>  DNA
<213>  Artificial

<220>
<223>  SfiI site


<220>
<221>  misc_feature
<222>  (5)..(9)
<223>  n is a, c, g, or t

<400>  1
ggccnnnnng gcc                                                          13


<210>  2
<211>  26
<212>  DNA
<213>  Artificial

<220>
<223>  nLIC fd primer

<400>  2
atggtgagaa tttatatttt caaggt                                            26


<210>  3
<211>  22
<212>  DNA
<213>  Artificial

<220>
<223>  nLIC rv primer

<400>  3
tgggagggtg ggattttcat ta                                                22


<210>  4
<211>  18
<212>  DNA
<213>  Artificial

<220>
<223>  cLIC fd primer

<400>  4
atgggtggtg gatttgct                                                     18

<210> 5
<211> 18
<212> DNA
<213> Artificial

<220>
<223> cLIC rv primer

<400> 5
ttggaagtat aaattttc 18


<210> 6
<211> 67
<212> DNA
<213> Artificial

<220>
<223> nLIC cassette


<220>
<221> CDS
<222> (1)..(48)

<400> 6
atg cat cat cac cat cat cac cat cac cat cat ggt gag aat tta tat 48
Met His His His His His His His His His His Gly Glu Asn Leu Tyr
1               5                   10                  15

ttaaatccca ccctcccag 67


<210> 7
<211> 16
<212> PRT
<213> Artificial

<220>
<223> Synthetic Construct

<400> 7

Met His His His His His His His His His His Gly Glu Asn Leu Tyr
1               5                   10                  15


<210> 8
<211> 50
<212> DNA
<213> Artificial

<220>
<223> SwaI digestion product 1 of nLIC cassette

<400> 8
atgcatcatc accatcatca ccatcaccat catggtgaga atttatattt 50


<210> 9
<211> 17
<212> DNA
<213> Artificial

<220>
<223> SwaI digestion product 2 of nLIC cassette

<400>  9
aaatcccacc ctcccag                                                    17


<210>  10
<211>  31
<212>  DNA
<213>  Artificial

<220>
<223>  nLIC after SwaI and T4 treatment


<220>
<221>  CDS
<222>  (1)..(30)

<400>  10
atg cat cat cac cat cat cac cat cac cat c                             31
Met His His His His His His His His His
1               5                   10


<210>  11
<211>  10
<212>  PRT
<213>  Artificial

<220>
<223>  Synthetic Construct

<400>  11

Met His His His His His His His His His
1               5                   10


<210>  12
<211>  29
<212>  DNA
<213>  Artificial

<220>
<223>  nLIC PCR(1)


<220>
<221>  CDS
<222>  (3)..(29)

<400>  12
at ggt gag aat tta tat ttt caa ggt atg                                29
   Gly Glu Asn Leu Tyr Phe Gln Gly Met
   1               5


<210>  13
<211>  9
<212>  PRT
<213>  Artificial

<220>
<223>  Synthetic Construct

<400>  13

Gly Glu Asn Leu Tyr Phe Gln Gly Met
1               5

<210>  14
<211>  22
<212>  DNA
<213>  Artificial

<220>
<223>  nLIC PCR(2)

<400>  14
taatgaaaat cccaccctcc ca                                                22


<210>  15
<211>  26
<212>  DNA
<213>  Artificial

<220>
<223>  PCR + T4 product (1)


<220>
<221>  CDS
<222>  (3)..(26)

<400>  15
at ggt gag aat tta tat ttt caa ggt                                      26
   Gly Glu Asn Leu Tyr Phe Gln Gly
   1               5


<210>  16
<211>  8
<212>  PRT
<213>  Artificial

<220>
<223>  Synthetic Construct

<400>  16

Gly Glu Asn Leu Tyr Phe Gln Gly
1               5


<210>  17
<211>  22
<212>  DNA
<213>  Artificial

<220>
<223>  PCR + T4 product (2)

<400>  17
tgggagggtg ggattttcat ta                                                22


<210>  18
<211>  57
<212>  DNA
<213>  Artificial

<220>
<223>  nLIC 5' end after annealing

<220>
<221> CDS
<222> (1)..(57)

<400> 18
atg cat cat cac cat cat cac cat cac cat cat ggt gag aat tta tat 48
Met His His His His His His His His His His Gly Glu Asn Leu Tyr
1               5                   10                  15

ttt caa ggt 57
Phe Gln Gly

<210> 19
<211> 19
<212> PRT
<213> Artificial

<220>
<223> Synthetic Construct

<400> 19

Met His His His His His His His His His His Gly Glu Asn Leu Tyr
1               5                   10                  15

Phe Gln Gly

<210> 20
<211> 17
<212> DNA
<213> Artificial

<220>
<223> nLIC 3' end after annealing (1)

<400> 20
aaatcccacc ctcccag 17

<210> 21
<211> 23
<212> DNA
<213> Artificial

<220>
<223> nLIC 3' end after annealing (2)

<400> 21
ctgggagggt gggattttca tta 23

<210> 22
<211> 68
<212> DNA
<213> Artificial

<220>
<223> cLIC cassette

<220>
<221> CDS
<222> (2)..(16)

<220>
<221>  CDS
<222>  (18)..(68)

<400>  22
c atg ggt ggt gga ttt a aat tta tac ttc caa ggt cat cat cac cat        47
  Met Gly Gly Gly Phe   Asn Leu Tyr Phe Gln Gly His His His His
  1               5                     10                  15

cat cac cat cac cat cat taa                                            68
His His His His His His
                20


<210>  23
<211>  5
<212>  PRT
<213>  Artificial

<220>
<223>  Synthetic Construct

<400>  23

Met Gly Gly Gly Phe
1               5


<210>  24
<211>  16
<212>  PRT
<213>  Artificial

<220>
<223>  Synthetic Construct

<400>  24

Asn Leu Tyr Phe Gln Gly His His His His His His His His His His
1               5                   10                  15


<210>  25
<211>  16
<212>  DNA
<213>  Artificial

<220>
<223>  cLIC after SwaI (1)

<400>  25
catgggtggt ggattt                                                      16


<210>  26
<211>  52
<212>  DNA
<213>  Artificial

<220>
<223>  cLIC after SwaI (2)

<400>  26
aaatttatac ttccaaggtc atcatcacca tcatcaccat caccatcatt aa              52


<210>  27

<211>  36
<212>  DNA
<213>  Artificial

<220>
<223>  cLIC after SwaI + T4

<400>  27
ttaatgatgg tgatggtgat gatggtgatg atgacc                                36


<210>  28
<211>  18
<212>  DNA
<213>  Artificial

<220>
<223>  cLIC PCR(1)


<220>
<221>  CDS
<222>  (1)..(18)

<400>  28
atg ggt ggt gga ttt gct                                                18
Met Gly Gly Gly Phe Ala
1               5


<210>  29
<211>  6
<212>  PRT
<213>  Artificial

<220>
<223>  Synthetic Construct

<400>  29

Met Gly Gly Gly Phe Ala
1               5


<210>  30
<211>  18
<212>  DNA
<213>  Artificial

<220>
<223>  cLIC PCR(2)


<220>
<221>  CDS
<222>  (1)..(18)

<400>  30
gaa aat tta tac ttc caa                                                18
Glu Asn Leu Tyr Phe Gln
1               5


<210>  31
<211>  6
<212>  PRT
<213>  Artificial

<220>
<223>  Synthetic Construct

<400>  31

Glu Asn Leu Tyr Phe Gln
1               5


<210>  32
<211>  19
<212>  DNA
<213>  Artificial

<220>
<223>  cLIC 5' end after annealing (1)


<220>
<221>  CDS
<222>  (2)..(19)

<400>  32
c atg ggt ggt gga ttt gct                                                19
  Met Gly Gly Gly Phe Ala
  1               5


<210>  33
<211>  6
<212>  PRT
<213>  Artificial

<220>
<223>  Synthetic Construct

<400>  33

Met Gly Gly Gly Phe Ala
1               5


<210>  34
<211>  16
<212>  DNA
<213>  Artificial

<220>
<223>  cLIC 5' end after annealing (2)


<220>
<221>  CDS
<222>  (2)..(16)

<400>  34
c atg ggt ggt gga ttt                                                    16
  Met Gly Gly Gly Phe
  1               5


<210>  35
<211>  5
<212>  PRT
<213>  Artificial

<220>
<223>  Synthetic Construct

<400>  35

Met Gly Gly Gly Phe
1               5


<210>  36
<211>  52
<212>  DNA
<213>  Artificial

<220>
<223>  cLIC 3' end after annealing (1)


<220>
<221>  CDS
<222>  (2)..(52)

<400>  36
a aat tta tac ttc caa ggt cat cat cac cat cat cac cat cac cat cat      49
  Asn Leu Tyr Phe Gln Gly His His His His His His His His His His
  1               5                   10                  15

taa                                                                    52


<210>  37
<211>  16
<212>  PRT
<213>  Artificial

<220>
<223>  Synthetic Construct

<400>  37

Asn Leu Tyr Phe Gln Gly His His His His His His His His His His
1               5                   10                  15


<210>  38
<211>  54
<212>  DNA
<213>  Artificial

<220>
<223>  cLIC 3' end after annealing (2)


<220>
<221>  CDS
<222>  (1)..(54)

<400>  38
gaa aat tta tac ttc caa ggt cat cat cac cat cat cac cat cac cat      48
Glu Asn Leu Tyr Phe Gln Gly His His His His His His His His His
1               5                   10                  15

cat taa                                                              54
His



<210>  39
<211>  17
<212>  PRT

<213>  Artificial

<220>
<223>  Synthetic Construct

<400>  39

Glu Asn Leu Tyr Phe Gln Gly His His His His His His His His His
1               5                   10                  15

His

<210>  40
<211>  13
<212>  DNA
<213>  Artificial

<220>
<223>  SfiI x

<400>  40
ggccactgag gcc                                                          13

<210>  41
<211>  13
<212>  DNA
<213>  Artificial

<220>
<223>  SfiI y

<400>  41
ggccggacgg gcc                                                          13

## Description

The invention relates to the field of molecular biology.

Micro-organisms are an attractive host for amplifying and expressing nucleic acid. Expression of nucleic acids which encode protein is a valuable tool for producing sufficient amounts of a protein of interest, such as for instance (therapeutic) enzymes. Well-established expression hosts are *E. coli* and yeast. However, proteins produced this way do not always have the native conformation. For instance, proteins are not always folded correctly by the micro-organisms that produce them and may contain unwanted post-translational modifications. Heterologous expression of large multidomain assemblies and membrane proteins in a functionally competent state is often problematic in the well-established expression hosts *E. coli* and yeast. Alternative expression systems are thus urgently needed to overcome this major hurdle in structural genomics projects. Frequently, efficient DNA manipulations form a bottleneck for exploring the protein expression potential of novel hosts, thereby preventing rapid and routine screening. Shuttle vectors that replicate both in *E. coli* and an alternative host offer a way out, but their use is complicated by the requirement for dual replication factors and multiple selection markers. This increases the plasmid size and often compromises stable propagation. For instance, *Lactococcus lactis* is widely recognized as an attractive alternative to *E. coli* and yeast-based expression systems. However, the expression screening in *L. lactis* has been seriously hampered by the low efficiency of gene manipulations in the organism and the instability of *L. lactis - E. coli* shuttle vectors. Additionally, the lack of an efficient cloning procedure has restricted the preparation of large gene libraries for directed evolution studies in *L. lactis;* similar limitations have prohibited the full exploitation of other micro-organisms as expression and screening hosts.

It is an object of the present invention to provide alternative means and methods for cloning a nucleic acid of interest in a micro-organism. Preferably, means and methods are provided for cloning a nucleic acid of interest in a micro-organism which is less amenable to conventional nucleic acid manipulations as compared to *E.coli.*

Accordingly, the present invention provides a method for cloning a nucleic acid sequence of interest in a first kind of micro-organism, the method comprising:
- inserting said nucleic acid of interest into a nucleic acid vehicle, which vehicle comprises an origin of replication of a second kind of micro-organism;
- cloning said vehicle in a culture of said second kind of micro-organism;
- isolating cloned vehicle comprising said nucleic acid sequence of interest;
- substituting the vehicle's origin of replication of a second kind of micro-organism by an origin of replication of said first kind of micro-organism; and
- cloning the resulting vehicle in a culture of said first kind of micro-organism.

With a method of the invention it has become possible to efficiently clone nucleic acid sequences of interest in micro-organisms that are less amenable to conventional nucleic acid manipulations, as compared to *E.coli.* The present invention enables high-throughput cloning (and, preferably, expressing) of a nucleic acid of interest in a first kind of micro-organism, while an initial (preferably high-throughput) cloning of the nucleic acid of interest is done in a second kind of micro-organism. Said second kind of micro-organism is preferably *E.coli.* The need of shuttle vectors is circumvented. With a method of the invention it has become possible to take advantage of the cloning properties of both said first and said second kind of micro-organism. Said second micro-organism is preferably a kind of micro-organism with a high cloning efficiency, such as *E.coli.* Said first kind of micro-organism preferably comprises an expression property of interest. Said first kind of micro-organism is for instance preferably capable of producing a protein of interest with a desired conformation.

In a first step of a method according to the invention a nucleic acid of interest is inserted into a nucleic acid vehicle, which vehicle comprises an origin of replication of a second kind of micro-organism. A nucleic acid vehicle is defined as a compound which comprises nucleic acid. Preferably, said nucleic acid vehicle consists of nucleic acid. The term "nucleic acid" encompasses sequences comprising natural nucleotides (adenine, guanine, cytosine, thymine and/or uracil) and/or non-natural nucleotides (such as for instance inosine). Artificial nucleic acid analogues, such as for instance - but not limited to - peptide nucleic acid (PNA), are also encompassed by the term "nucleic acid".

Said nucleic acid vehicle preferably comprises a plasmid or a vector. In this first step, use is made of a nucleic acid vehicle which comprises an origin of replication of a second kind of micro-organism. Said origin of replication is a nucleic acid region which is necessary for initiation of replication of the nucleic acid vehicle. An origin of replication of a second kind of micro-organism is recognized by the nucleic acid replication machinery of said second kind of micro-organism. Different micro-organisms require different kinds of replication origins; an origin-recognition protein of a micro-organism (e.g. DnaA in *E.coli*) is capable of binding to an origin of replication. Upon recognition, replication is started, which require additional proteins that can be specific for a given micro-organism. Hence, an origin of replication has to be chosen which is recognized by a micro-organism of choice. If *E.coli* is chosen as a second kind of micro-organism, the *E.coli* origin of replication from pBR322 is for instance used. However, any origin of replication of a micro-organism of choice is suitable.

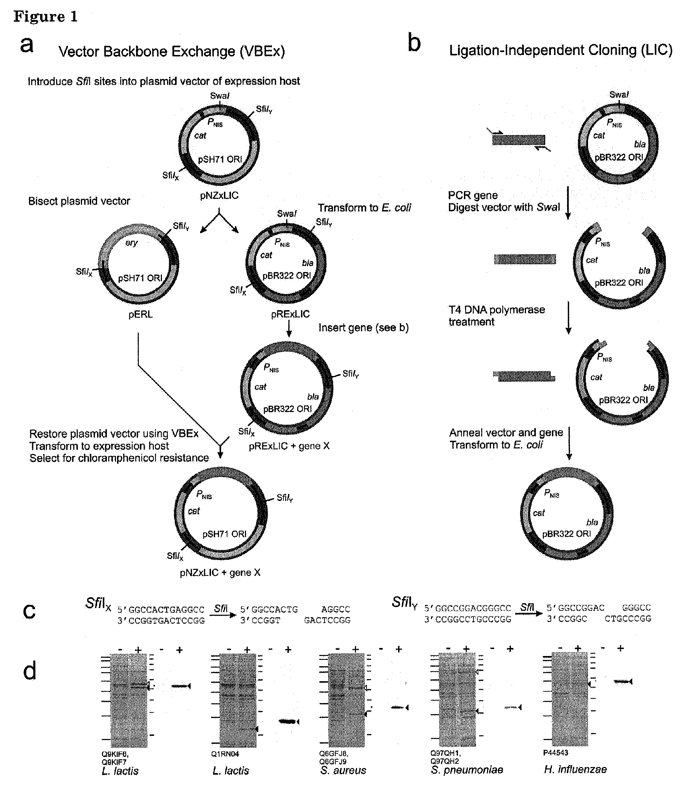

A nucleic acid of interest is inserted into a nucleic acid vehicle using any cloning method available in the art. In a preferred embodiment, said nucleic acid of interest is inserted into said first vehicle and cloned in a culture of said second kind of micro-organism using a method selected from the group consisting of a Ligation Independent Cloning (LIC) procedure, Gateway, Univector Plasmid-fusion System (UPS), and methods that are derived from LIC such as for instance Enzyme-Free Cloning (EFC) or Sequence and Ligation Independent Cloning (SLIC). In one embodiment a ligation-independent cloning (LIC) procedure (Aslanidis and de Jong, 1990) is used. Contrary to methods that rely on recombination events (e.g., Gateway (Walhout et al, 2000) or the Univector Plasmid-fusion System (UPS) (Liu et al, 1998)), ligation-independent cloning is less restricted in the design of the sequences flanking the gene(s). Therefore, the cloning-related sequences attached to the recombinant protein(s) can be minimized. One embodiment of a LIC procedure is schematically outlined in Figure 1B. This embodiment of a LIC cloning procedure involves linearization of a vector by restriction at a unique *Swa*I site in the middle of the LIC cassette, followed by T4 DNA polymerase treatment to create long, defined single-stranded overhangs, complementary to those of a nucleic acid sequence (for instance a PCR product). The resulting vector and nucleic acid sequence are mixed and transformed to an expression host, for instance by chemical transformation using CaCl₂. The LIC cassettes are preceded by promoter regions specific for the intended expression host. Ligation-independent cloning of nucleic acid products proved highly efficient for *E. coli* vectors, indicating that the length and composition of the complementary overhangs of vector and insert suffice for formation of stable heteroduplexes. In contrast, direct transformation of *L. lactis* with a stable heteroduplex of a LIC vector and a compatible insert yielded no or only very few positive clones. Therefore, a second micro-organism is preferably chosen which allows for efficient cloning, such as - but not limited to - *E. coli.*

In a second step of a method according to the invention, cloned vehicle comprising a nucleic acid of interest is isolated. This is done using any nucleic acid isolation method known in the art, such as for instance the method of Birnboim and Doly (1979).

Subsequently, the origin of replication of the cloned vehicle is substituted by an origin of replication of a first micro-organism. This is preferably done using a recombinase or a restriction enzyme. In that case the vehicle is designed such that a first recombinase site or restriction enzyme recognition sequence is present before the origin of replication of a second micro-organism. A second recombinase site or restriction enzyme recognition sequence is present after said origin of replication. Upon incubation with a recombinase or a restriction enzyme recognizing said sequences, the origin of replication is cut out of the vehicle. Subsequently, an origin of replication of a first micro-organism is inserted into the vehicle. The resulting vehicle is preferably essentially devoid of elements derived from said second kind of micro-organism in order to allow efficient cloning and maximal stability of the vehicle in said first kind of micro-organism. Hence, with a method according to the invention, the use of shuttle vectors is not necessary. This is advantageous because any additional sequence increases the size of the resulting vector. In order to be capable of efficient cloning, the size of the resulting vehicle is preferably as small as possible. Moreover, smaller vehicles are more stable. Hence, the presence of additional, foreign sequences unnecessarily enlarges the resulting vehicle, decreases the stability and decreases the maximum size that a nucleic acid sequence of interest is allowed to have. An embodiment of the present invention wherein the resulting vehicle is essentially devoid of elements derived from said second kind of micro-organism is therefore preferred. By essentially devoid is meant that the resulting vehicle comprises no more than 15, preferably no more than 10 nucleotides derived from said second kind of micro-organism. Moreover, the resulting vehicle preferably comprises no more than 100, more preferably no more than 50, most preferably no more than 30 "foreign" nucleotides originating from the recombinase recognition sites and/or restriction enzyme recognition sites of the original vehicle(s).

A non-limiting, preferred embodiment is schematically shown in figure 1A. According to this embodiment, a nucleic acid of interest is inserted into a first vehicle comprising an *E.coli* origin of replication. Said vehicle is amplified in *E.coli.* Subsequently, the vector is isolated and cut by the *Sfi*I restriction enzyme, preferably in the presence of another vector comprising an *L.lactis* origin of replication which is flanked by similar S*fi*I sites. Upon treatment with T4 DNA ligase and ATP, the *E.coli* origin of replication of the first vehicle is subsequently replaced by the *L.lactis* origin of replication in a significant part of the vehicles. Of course, many alternative embodiments are within the scope of the present invention. Any way of exchanging any origins of replication is suitable.

In a subsequent step of a method according to the invention, the resulting nucleic acid vehicles comprising a nucleic acid sequence of interest and an origin of replication of a first micro-organism are transformed in said first kind of micro-organism. Said nucleic acid sequence of interest is preferably expressed in said first kind of micro-organism. This way, efficient cloning and expression of a nucleic acid of interest in any micro-organism for which plasmids are available has become feasible. Since a method of the invention allows the use of a second kind of micro-organism more amenable to (high-throughput) cloning, the cloning efficiency limitations of many first kinds of micro-organisms are at least in part bypassed.

In a preferred embodiment at least two nucleic acid vehicles are used. One vehicle comprises an origin of replication of a first kind of micro-organism and one vehicle comprises an origin of replication of a second kind of micro-organism. A nucleic acid sequence of interest is inserted into the vehicle comprising an origin of replication of a second kind of micro-organism and the resulting vehicle is transformed in said second kind of micro-organism. Subsequently, the origin of replication of the vehicle containing the nucleic acid sequence of interest is replaced by the origin of replication of the other vehicle and the resulting vehicle is subsequently transformed in the first kind of micro-organism. A preferred embodiment thus provides a method according to the invention comprising:
- providing a first nucleic acid vehicle which comprises an origin of replication of said second kind of micro-organism;
- introducing a nucleic acid sequence of interest into said first vehicle;
- providing a second nucleic acid vehicle which comprises an origin of replication of said first kind of micro-organism; and
- substituting a part of said first vehicle, which part comprises said origin of replication of said second micro-organism but not said nucleic acid of interest, by a part of said second vehicle, said part comprising said origin of replication of said first microorganism.

Preferably said second vehicle comprises a complete origin of replication of said first kind of micro-organism. In one alternative embodiment said second vehicle comprises a part of an origin of replication of said first kind of micro-organism. In this embodiment, said first vehicle preferably also comprises a part of said origin of replication of said first kind of micro-organism, which part is preferably not present in said second vehicle. After a nucleic acid of interest has been inserted into said first vehicle, a sequence of said second vehicle comprising said part of an origin of replication of said first micro-organism is preferably inserted into said first vehicle. In one embodiment a part of said origin of replication of said first micro-organism which is present in said first vehicle and a part of said origin of replication of said first micro-organism which is present in said second vehicle are functionally linked together, so that said parts are capable of acting together. Said second vehicle preferably comprises a significant part, more preferably a functional part, of said origin of replication of said first kind of micro-organism.

The invention also provides means and methods for even further optimizing nucleic acid manipulation efficiency. The present inventors have recognized that the use of restriction enzymes that produce different, non-palindromic overhangs that are not compatible with each other is preferred in order to enhance cloning efficiency. Non-limiting examples of such restriction enzymes are *Sfi*I, *SapI*, *Ksp632I, AarI, BglI, PflMI and BstXI.* Said restriction enzymes preferably recognize nucleic acid sequences that rarely occur in the genomes of prokaryotes and eukaryotes, so that unintentional cutting of nucleic acid inserts of interest is best avoided. In principle, the more specific base pairs a recognition site has, the smaller the chance is that it occurs in a given nucleic acid sequence. The recognition sites of *Ksp632I, BglI, PflMI and BstXI* have six specific base pairs, the recognition sites of *SapI* and *AarI* have seven specific base pairs and the recognition site of *SfiI* has eight specific base pairs. Therefore, in order to even better avoid unintentional cutting of nucleic acid insert sequences of interest, a method according to the invention is preferably performed using *SapI, AarI* or *SfiI.* Most preferably, *Sfi*I is used. Furthermore, a restriction enzyme that is active in the same kind of buffer wherein T4 DNA ligase is active is preferred, so that in one embodiment cleavage reactions and ligase reactions with T4 DNA ligase are carried out without need to change the reaction buffer, mere supplementation of the buffer with T4 ligase and ATP would suffice to start the ligation. *Sfi*I is active in the same kind of buffer wherein T4 DNA ligase is active. Therefore, *Sfi*I is also preferred because of this property. By compatible is meant that two nucleic acid sequences are capable of binding each other because they comprise single stranded complementary sequences of a sufficient length. Hence, nucleic acid sequences that are not compatible with each are not capable of annealing to each other because they do not comprise single stranded, complementary sequences of a sufficient length.

A particularly preferred restriction enzyme is *Sfi*I. *Sfi*I recognizes the sequence 5' GGCCNNNNNGGCC 3' (N being any nucleotide). The sequence is cut at:
5' GGCCNNNN ^ NGGCC 3'
3' CCGGN ^ NNNNCCGG 5'

As a result, an overhang of NNN is produced. Hence, as indicated in Figure 1C, nucleic acid cleavage by *Sfi*I generates a 3' overhang that can be composed of any combination of three nucleotides. In a preferred embodiment of a method of the present invention, a first nucleic acid vehicle is used which comprises preferably two different *Sfi*I sites, flanking an origin of replication of a second kind of micro-organism, which *Sfi*I sites, upon incubation with *Sfi*I*,* yield different, non-palindromic overhangs that are not compatible with each other. Incubation with *Sfi*I results in a cut vehicle which is devoid of the origin of replication of said second micro-organism. Two ends of this vehicle that has been cut by *Sfi*I do not anneal to each other. According to a further preferred embodiment, said two ends are capable of annealing to a second nucleic acid comprising a different origin of replication. This way, said second nucleic acid is very efficiently introduced into said vehicle.

Said second nucleic acid comprising a different origin of replication preferably originates from a second nucleic acid vehicle comprising said origin of replication, flanked by *Sfi*I sites. The *Sfi*I sites are chosen such that the resulting overhangs are compatible with the *Sfi*I overhangs of the above mentioned vehicle that has been cut by *Sfi*I. Upon incubation with *Sfi*I, the second vehicle is cut and a nucleic acid sequence is obtained which comprises an origin of replication of a first micro-organism with *Sfi*I overhangs capable of annealing to said first vehicle which has been cut by *Sfi*I.

A preferred embodiment thus provides a method for cloning a nucleic acid sequence of interest in a first kind of micro-organism, the method comprising:
- providing a first nucleic acid vehicle which comprises an origin of replication of said second kind of micro-organism;
- introducing a nucleic acid sequence of interest into said first vehicle;
- providing a second nucleic acid vehicle which comprises an origin of replication of said first kind of micro-organism; and
- substituting a part of said first vehicle, which part comprises said origin of replication of said second micro-organism but not said nucleic acid of interest, by a part of said second vehicle, said part comprising said origin of replication of said first microorganism, wherein said part of said first vehicle has different, non-palindromic overhangs that are not compatible with each other and that are compatible with the overhangs of said part of said second vehicle.

Said parts are preferably obtained by cleavage of said vehicles by *Sfi*I.

A method of the invention involves transformation of nucleic acid sequences of interest to micro-organisms. In order to select micro-organisms that comprise the nucleic acid sequences of interest, selection markers are preferably used. Preferably, a nucleic acid sequence comprising an origin of replication of a first kind of micro-organism together with a first selection marker is used. Additionally, or alternatively, a nucleic acid sequence comprising an origin of replication of a second kind of micro-organism together with a second selection marker is preferably used. A non-limiting example of a suitable selection marker is a gene which provides resistance against the action of an enzyme and/or antibiotic, such as for instance a chloramphenicol resistance gene or a betalactam antibiotic resistance gene. Alternatively, or additionally, a selection marker is used that complements an auxotrophy (e.g. biosynthetic deficiency) in the microorganism in which the nucleic acid sequences are preferably expressed, such as for instance the alr gene, encoding an alanine racemase (Bron et al, 2002), or allows selection based on alternative selection procedures, such as for instance the *shsp* gene, encoding a small heat shock protein (El Demerdash et al, 2003) If a nucleic acid sequence is coupled to a selection marker, the presence of said nucleic acid sequence in a micro-organism is determined by determining whether the selection marker is present. For instance, an origin of replication of a second kind of micro-organism is coupled to a beta-lactamase resistance gene. After transformation, the resulting micro-organisms are propagated in the presence of beta-lactamase. Micro-organisms that are propagated apparently carry the beta-lactamase resistance gene which indicates that they also carry the origin of replication of a second kind of micro-organism.

A non-limiting example of the use of selection markers is schematically depicted in Figure 1A. A vector comprising an *E.coli* origin of replication is provided with a nucleic acid sequence of interest and transformed in *E.coli.* This vector has both a chloramphenicol resistance gene (cat) and a beta-lactamase resistance gene (bla). *E.coli* micro-organisms carrying this vector are resistant to beta-lactamase. Subsequently, the *E.coli* origin of replication is replaced by an *L.lactis* origin of replication. The resulting vector does no longer comprise the beta-lactamase resistance gene, but does contain the *L.lactis* origin of replication. Hence, *L.lactis* clones that are resistant to chloramphenicol contain a nucleic acid vehicle containing both the chloramphenicol resistance gene and the *L.lactis* origin of replication which allows propagation of the nucleic acid in *L.lactis.* Of course, many other (combinations of) selection markers are suitable for selecting micro-organisms of interest.

Further provided is therefore a method for cloning a nucleic acid sequence of interest in a first kind of micro-organism, the method comprising:
- providing a first nucleic acid vehicle which comprises an origin of replication of said second kind of micro-organism;
- introducing a nucleic acid sequence of interest into said first vehicle;
- providing a second nucleic acid vehicle which comprises an origin of replication of said first kind of micro-organism; and
- substituting a part of said first vehicle, which part comprises said origin of replication of said second micro-organism but not said nucleic acid of interest, by a part of said second vehicle, said part comprising said origin of replication of said first microorganism, wherein said part of the first vehicle comprising an origin of replication of said second micro-organism comprises a selection marker, such as for instance a resistance gene. In one embodiment said second vehicle comprising an origin of replication of said first micro-organism comprises a different selection marker, such as for instance a different resistance gene.

A method according to the present invention is particularly suitable for improving the efficiency of cloning and expressing a nucleic acid sequence of interest in a micro-organism of interest. Preferably, a first cloning step is performed wherein a nucleic acid of interest is efficiently inserted into a nucleic acid vehicle and cloned in another kind of micro-organism which has better cloning properties as compared to the micro-organism wherein the nucleic acid of interest is finally expressed. One embodiment thus provides a method for cloning a nucleic acid sequence of interest in a first kind of micro-organism, the method comprising:
- providing a first nucleic acid vehicle which comprises an origin of replication of a second kind of micro-organism;
- introducing a nucleic acid sequence of interest into said first vehicle;
- cloning said vehicle in a culture of said second kind of micro-organism;
- providing a second nucleic acid vehicle which comprises an origin of replication of said first kind of micro-organism;
- substituting a part of said first vehicle, which part comprises said origin of replication of said second micro-organism but not said nucleic acid of interest, by a part of said second vehicle, said part comprising said origin of replication of said first microorganism; and
- cloning the resulting vehicle in a culture of said first kind of micro-organism;
wherein the process of inserting said nucleic acid of interest into said first vehicle and cloning it in a culture of said second kind of micro-organism is more efficient as compared to a process of inserting said nucleic acid of interest into said second vehicle and cloning it in a culture of said first kind of micro-organism.

Said second micro-organism is preferably *Escherichia coli,* because this micro-organism is known to have high transformation efficiencies and various gene manipulation tools have been specifically developed and optimized for this organism. This is amongst other things due to the fact that suitable plasmid systems exist in the art. Insertion of nucleic acids of interest into such plasmid systems and cloning of the resulting plasmids have proven to be very efficient in *E.coli.* This micro-organism is therefore very suitable for use in a first cloning step of a method according to the invention.

In one embodiment said first micro-organism is *Lactococcus lactis, Streptomyces* sp or *Sulfolobus solfataricus.* These micro-organisms are preferred because various proteins are better expressed by these micro-organisms as compared to *E.coli.* For instance, various proteins obtain a better conformation and/or post-translational modification(s) as a result of the cellular environment of these other micro-organisms, as compared to *E.coli.* Moreover, additional parametes such as their codon usage, types and quantities of chaperones, resistance to expression of proteins which are toxic to *E*. *coli,* and lipid composition of the plasma membrane is often more favorable to protein expression in a functional state as compared to *E.coli.* Micro-organisms other than *E.coli* are also preferred for expressing proteins if the produced proteins are more stable in their close to own cellular environment. Hence, even though a conventional cloning procedure such as a LIC procedure is much less efficient in *L.Lactis, Streptomyces* sp or *S. solfataricus,* as compared to *E.coli,* it is often still preferable to clone and express a nucleic acid sequence in *L.lactis, Streptomyces* sp or *S. solfataricus.* With a method according to the invention, efficient cloning and expression of nucleic acid sequences in such recalcitrant micro-organisms has become possible.

A method according to the invention is particularly suitable for efficient cloning and expressing a nucleic acid sequence of interest by a micro-organism of choice. A method according to the invention, further comprising allowing expression of said nucleic acid of interest by a culture of said first kind of micro-organism is therefore also herewith provided. Once a nucleic acid of interest is expressed in a micro-organism with a method according to the invention, such expression product is preferably obtained for further use. For instance, a therapeutic protein is produced, isolated and used for the preparation of a medicament. Many methods for obtaining, isolating and/or purifying protein that has been produced by micro-organisms are known in the art. Non-limiting examples include the use of affinity tags such as oligo-His-tags in combination with metal-chelate affinity chromatography, MBP-tags in combination with amylose-resin affinity chromatography and other generic chromatography methods to purify proteins. A method according to the invention, further comprising obtaining an expression product of said nucleic acid sequence of interest is therefore also herewith provided, as well as an expression product obtained by a method according to the invention.

In one embodiment said nucleic acid of interest encodes a multidomain protein and/or a membrane protein. Heterologous expression of such proteins in a functionally competent state is often problematic in the well-established expression hosts *E.coli* and yeast. With a method of the invention, however, efficient cloning and expression of nucleic acid sequences encoding such proteins in alternative expression hosts has become possible.

Now that it has become possible to efficiently clone and express nucleic acid sequences in a wide variety of micro-organisms, a method of the invention is performed for many applications. For instance, nucleic acid sequences are mutated (for instance using error-prone PCR) and the resulting nucleic acid sequences are cloned and expressed. High-throughput screening of mutated proteins in order to look for improved variants has become possible. Micro-organisms other than the well-established *E.coli* are now used for high-throughput cloning and expression. Mutant proteins that do not obtain a desired conformation or post-translational modification in *E.coli* are now efficiently expressed using other kinds of micro-organisms. A cloning assay wherein a nucleic acid of interest is cloned and expressed by a method according to the invention is therefore also provided. Said cloning assay is preferably a high-throughput cloning assay.

Further provided is a kit of parts comprising:
- a first nucleic acid vehicle which comprises an origin of replication of a second kind of micro-organism; and
- a second nucleic acid vehicle which comprises an origin of replication of a first kind of micro-organism;
wherein each vehicle comprise at least two recombinase sites and/or restriction enzyme cleavage sites wherein said restriction enzyme cleavage sites are preferably not present within said origins of replication.

This way, the origins of replication are cut out of the vector and exchanged by the other kind of origin of replication at will.

Preferably, each vehicle comprise at least two restriction enzyme cleavage sites which, upon cleaving, yield two different, non-palindromic overhangs that are not compatible with each other so that two ends of a vehicle that has been cut do not anneal to each other. This way, cloning efficiency is even further enhanced. When non-complementary, non-palindromic overhangs are used, a nucleic acid sequence of interest is in principle only capable of being inserted into a nucleic acid vehicle in one direction. Non-complementary, non-palindromic overhangs are therefore preferred in order to obtain a nucleic acid vehicle with a nucleic acid sequence of interest in the right orientation.

In one embodiment said first nucleic acid vehicle comprises a sequence encoding an affinity tag (such as for instance a His-tag) and/or another kind of fusion partner (such as for instance GFP), which affinity tag or fusion partner will become attached to the protein(s) encoded by the nucleic acid sequence of interest. Such affinity tag or fusion partner facilitates isolation, and/or detection, and/or purification of said protein(s).

Such kit of parts is particularly suitable for performing a method according to the invention, wherein a nucleic acid sequence of interest is introduced into said first vehicle, where after the resulting vehicle is cloned in a culture of said second kind of micro-organism, where after the cloned vehicle's origin of replication is replaced by the origin of replication of said second vehicle, where after the resulting vehicle is cloned in a culture of said first kind of micro-organism. Said origins of replications are preferably separated from the vehicles where they are originally present by the action of a recombinase or one or more restriction enzymes. Therefore, recombinase sites and/or restriction enzyme cleavage sites are preferably present in the flanking regions of said origins of replication.

A kit of parts according to the invention preferably comprises a first and a second vehicle, wherein each vehicle comprises the same kind of restriction enzyme cleavage sites which, upon cleaving, yield two different, non-palindromic overhangs that are not compatible with each other so that two ends of a vehicle that has been cut do not anneal to each other. This way, cloning efficiency is even further enhanced. The restriction enzyme sites of the vehicles are preferably chosen such that, upon incubation with said restriction enzyme, the parts of the vehicles that are intended to be ligated to each other have complementary single stranded overhangs. Preferably, at least one vehicle of a kit of parts according to the invention has at least two *Sfi*I cleavage sites. Most preferably, all vehicles of a kit of parts according to the invention have at least two *Sfi*I cleavage sites.

One preferred embodiment provides a kit of parts according to the invention, wherein said first vehicle comprises an *Escherichia coli* origin of replication. As already outlined before, *E.coli* is particularly suitable for the first cloning step of a method according to the invention. In one embodiment said *Escherichia coli* origin of replication is derived from pBR322.

The second vehicle of a kit of parts according to the invention preferably comprises a *Lactococcus lactis* origin of replication, a *Streptomyces* sp origin of replication or an *S.solfataricus* origin of replication. These micro-organisms are preferred for the production of various kinds of (human) proteins, as discussed before. In one embodiment said *Lactococcus lactis* origin of replication is derived from pSH71.

The first vehicle of a kit of parts according to the invention preferably comprises a selection marker, such as for instance an antibiotic resistance gene. Furthermore, the second vehicle of said kit of parts preferably comprises a selection marker, such as for instance an antibiotic resistance gene. Selection markers are used for selection of micro-organisms which carry the nucleic acid vehicle, as explained herein before.

With a method according to the invention it has become possible to efficiently clone a nucleic acid of interest using a kind of micro-organism which is less amenable to conventional nucleic acid manipulations. For instance, a Lactococcus lactis culture has been obtained wherein the amount of colony forming units per microgram nucleic acid which was used in said method is at least 10⁵ CFU/µg DNA.

Further provided is therefore a *Lactococcus lactis* culture obtainable by a method according to the invention, wherein the amount of colony forming units per microgram nucleic acid of interest which was used in said method is at least 10⁵ CFU/µg DNA.

The invention also provides a recombinant micro-organism comprising a nucleic acid vehicle, which vehicle comprises:
- an origin of replication of said kind of micro-organism;
- a nucleic acid sequence of interest; and
- at least two restriction enzyme cleavage sites which, upon cleaving, yield two different, non-palindromic overhangs that are not compatible with each other and
wherein said restriction enzyme cleavage sites are not present within said origins of replication. Said micro-organism is preferably *Lactococcus lactis.*

Moreover, said restriction enzyme cleavage sites are preferably *Sfi*I cleavage sites. As described herein before, the use of *Sfi*I allows cleavage of a nucleic acid vehicle, whereby two ends of a vehicle that has been cut do not anneal to each other. *Sfi*I cleavage sites are designed such that the parts of two different vehicles that are intended to be ligated to each other have complementary single stranded overhangs. The use of *Sfi*I further provides the additional advantage that it is active in the same kind of buffer wherein T4 DNA ligase is active. Hence, in a preferred embodiment wherein T4 DNA ligase and *Sfi*I are used, the cleavage reactions and ligase reactions are carried out without need to change the reaction buffer (meaning that actions other than the administration of *Sfi*I, T4 DNA ligase and ATP are not necessary).

The invention is further explained in the following examples. These examples do not limit the scope of the invention, but merely serve to clarify the invention.

### Examples

We now present a generic cloning strategy, compatible with high-throughput manipulations, that generates a native plasmid vector optimal for the expression host and devoid of alien (e.g., *E. coli*-derived) elements. The Vector Backbone Exchange (VBEx) procedure presented here is specific for cloning in *L*. *lactis* but readily adaptable for all organisms for which a plasmid, selection marker and transformation method are available. VBEx has been used to generate over 300 gene constructs, and gene cloning up to expression screening in *L. lactis* has been carried out at a rate of 48 per week.

To facilitate the initial steps in the cloning of large numbers of open reading frames, we employed a ligation-independent cloning (LIC) procedure (Aslanidis and de Jong, 1990). Contrary to methods that rely on recombination events (e.g., Gateway (Walhout et al, 2000) or the Univector Plasmid-fusion System (UPS) (Liu et al, 1998)), ligation-independent cloning is less restricted in the design of the sequences flanking the gene(s). Therefore, the cloning-related sequences attached to the recombinant protein(s) can be minimized. The cloning procedure involves linearization of the vector by restriction at a unique *Swa*I site in the middle of the LIC cassette, followed by T4 DNA polymerase treatment to create long, defined single-stranded overhangs, complementary to those of the PCR product (Fig. 1B). The LIC cassettes are preceded by promoter regions specific for the intended expression host. Here, we used the *P*_{BAD} and *P*_{NIS} promoters of the *E*. *coli* and *L. lactis* expression plasmids pBAD24 (Guzman et al, 1995) and pNZ8048 (de Ruyter et al, 1996), respectively. Ligation-independent cloning of PCR products proved highly efficient for the *E*. *coli* vectors, indicating that the length and composition of the complementary overhangs of vector and insert suffice for formation of stable heteroduplexes. In contrast, direct transformation of *L. lactis* with the stable heteroduplex of the pNZ8048-derived LIC vector and a compatible insert yielded no or only very few positive clones (data not shown).

To overcome the poor cloning efficiencies in *L. lactis,* a new strategy (VBEx) was devised that allowed the initial (high-throughput) cloning to be done in *E. coli,* but avoided the use of shuttle vectors. Although we have used VBEx in combination with LIC, the strategy is fully compatible with Gateway, UPS and variations on LIC (e.g, Enzyme Free Cloning (de Jong et al, 2007) and SLIC (Li and Elledge, 2007)). The method relies on the bisection of a *bona fide* plasmid vector of the expression host into two parts, thereby separating the selection marker and the origin of replication. For *L. lactis,* the origin of replication of the pNZ8048 plasmid (pSH71 replicon) was separated from the chloramphenicol resistance gene (*cat*) (Fig. 1a). The segment containing the *cat* and LIC sequence was fused to the backbone of a vector containing an *E. coli* origin of replication and the β-lactamase resistance gene (*bla*)*.* The resulting plasmid, pRExLIC, allows the LIC manipulation to take place in *E. coli.* The segment containing the *L. lactis* origin of replication was fused to an erythromycin selection marker (yielding plasmid pERL), which enables replication and selection in the expression host.

Rapid and high-throughput compatible reconstruction of the original *L.lactis* expression plasmid from the relevant segments of pRExLIC and pERL is assured by flanking the ends of each half with distinct *Sfi*I restriction sites (*Sfi*Ix and *Sfi*I_{Y} in Fig. 1a). DNA cleavage by *Sfi*I generates a 3' overhang that can be composed of any combination of three nucleotides. The two *Sfi*I sites used, yield different, non-palindromic overhangs that are not compatible with each other (Fig. 1c). The combination of *i)* both halves of the original expression vector having properties that can be selected for, and *ii)* different 3' overhangs after *Sfi*I digestion at either side of each plasmid segment, ensures minimal sample handling and allows automation of the method. As each half of the expression vector has unique selectable properties, no gel electrophoresis and purification of the *Sfi*I-fragments is required. Selection for the ability of the plasmid to replicate in *L. lactis* in the presence of chloramphenicol permits unique recovery of only the original expression vector from a complex mixture of *Sfi*I-digested pRExLIC and pERL plasmids. Furthermore, no change of buffers between the digestion and ligation reaction is needed. Mere supplementation of the *Sfi*I-buffer with ATP suffices for the T4 DNA ligase to be fully active.

In practice, the whole procedure can take place in a single tube in 3 hours. After joined *Sfi*I digestion of pRExLIC and pERL (80 min at 50°C), and subsequent heat inactivation of the restriction enzyme (20 min at 80°C), ligation (60 min at 20°C) of the fragments is started by the addition of ATP and T4 DNA ligase. Upon thermal inactivation of the T4 DNA ligase (20 min at 65°C), aliquots of the mixture can be used for (electro-)transformation without further purification (Fig. 1). For *L. lactis,* we routinely observe high, reproducible transformation efficiencies (~10⁶ CFU/µg DNA), in comparison to the low, variable efficiencies of traditional restriction-ligation cloning (~10² CFU/µg DNA; e.g. see Surade et al, 2006)). Notably, with the VBEx procedure, all *L. lactis* transformants obtained carry the gene of interest inserted in pNZxLIC. Using this system, we generated over 300 expression constructs and assessed protein levels at a rate of 48 constructs per week without robotics. The overexpression of a small subset of membrane proteins in *L. lactis* is presented in Fig. 1d.

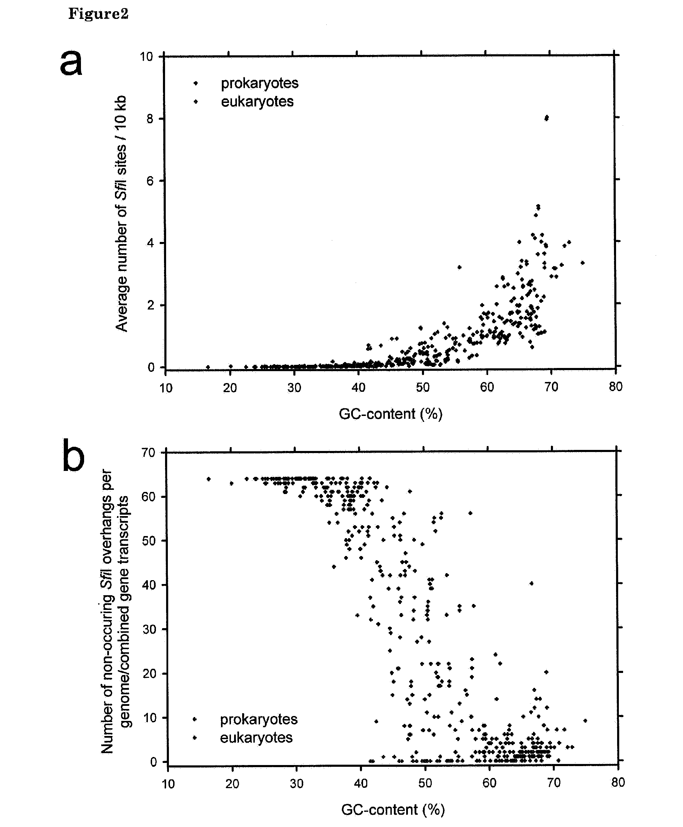

Importantly, DNA sequences from virtually all sequenced genomes are compatible with the cloning strategy, because *Sfi*I sites (5' GGCCNNNN^NGGCC 3') are rare (Fig 2a). Analysis of all predicted gene transcripts of 492 archaeal and bacterial chromosomes and 30 eukaryotic genomes, present in the NCBI databank (February 2007) and Ensembl (release 43), respectively, indicated that well over 92% of these transcripts do not contain any *Sfi*I site (Table 1). Moreover, use of the method is not limited to transcripts free of *Sfi*I sites. As 64 different 3' overhangs may be generated after *Sfi*I digestion, internal *Sfi*I sites with 3' overhangs not matching those of the vector will result in a three or more-way ligation, but not form a bottleneck for the procedure. If needed, the vector can be readily adapted to use non-occuring or extremely rare overhangs. In 89% of the genomes analyzed at least two types of *Sfi*I overhangs do not occur (Fig 2b). The remaining 11% of the genomes contain several types of low occurrence *Sfi*I overhangs.

To ensure the generality of the presented strategy towards inserts of different size, we compared *Sfi*I digestion rates of pRExLIC derivatives holding inserts up to 3.7 kb (data not shown) and observed complete digestion after 80 min incubation. Furthermore, we demonstrated the absence of expression from the *P*_{NIS} promoter in the cloning host *E*. *coli,* using a sensitive activity based assay (data not shown). The success of assembly and stability in the pRExLIC and pNZxLIC vectors of recombinant DNA from bacterial, plant and mammalian origin proved very high and gene rearrangements have so far not been observed (n >300).

In summary, we have developed LIC-VBEx, a high-throughput compatible cloning system for *L. lactis* with unprecedented high efficiency, which can be readily adapted for any other expression host. Problems arising from shuttle vectors are avoided by using genuine expression plasmids. The LIC cassettes developed allow the tagging of the protein of interest with a cleavable decaHistag at either the N- or C-terminus (vectors holding these cassettes and derivatives with alternative tags or fusion-partners are shown in Materials and Methods section). The procedure described has thus far been used in our lab to prepare over 300 gene constructs with high efficiencies (~90% for LIC; 100% for VBEx (data not shown)). In a non-automated setting, the full procedure from cloning to expression screening in *E. coli* and *L. lactis,* took place at a rate of approximately 48 constructs per week.

### Materials and methods

**Ligation-Independent Cloning (LIC)**. Inserts were amplified using Phusion DNA polymerase (Finnzymes) and gene specific primers extended at the 5' side with LIC specific tails (Table A). Vectors containing a LIC cassette were purified using a plasmid isolation kit (Wizard ® Plus, Promega). DNA was additionally purified by extraction with phenol:chloroform and chloroform to remove tracer amounts of proteins. Vectors (~5 µg of DNA) were digested overnight with 25 units of *Swa*I (Roche) at 25°C. PCR products and digested vectors were subsequently gel purified (GFX PCR DNA & Gel Band Purification Kit, GE), eluted in 10 mM Tris-HCl, pH 7.5, 0.2 mM Na-EDTA, and stored at 4°C.

200 ng of SwaI-digested vector and equimolar quantities of inserts were treated separately with 0.5 U T4 DNA polymerase (Roche) at 20°C for 30 min in the presence of 2.5 mM dCTP and 2.5 mM dGTP, respectively, followed by heat inactivation of the T4 DNA polymerase (20 min at 75°C). The material, now in a "LIC-ready" state, can be stored at 4°C for prolonged periods (over 6 months). LIC-ready vector (1 µl) and insert (3 µl) were mixed and after a 5 min incubation at RT transformed to 75 µl chemically-competent *E. coli* MC1061. Cells were plated on Luria Broth supplemented with ampicillin (100 µg / ml).

**Table A. Primer extensions required for Ligation Independent Cloning.**

| **Type of primer** | **Sequence (5'→3')** |
|---|---|
| nLIC forward | AT GGT GAG AAT TTA TAT TTT CAA GGT + gene specific |
| nLIC reverse | T GGG AGG GTG GGA TTT TCA TTA + gene specific |
| cLIC forward | ATG GGT GGT GGA TTT GCT + gene specific |
| cLIC reverse | TTG GAA GTA TAA ATT TTC + gene specific |

**Vector Backbone Exchange.** Exchange of the vector backbone of pRExLIC-derived vectors was done in a small volume (10 µl) in a PCR machine with heated lid to avoid condensation. The pERL vector (containing the *L. lactis* origin of replication) and a pRExLIC-derived vector (containing the *L. lactis cat* gene) were mixed (~125 ng each) and the volume was adjusted to 10 µl by adding 1 µl 10 X buffer (100 mM Tris-HCl, pH 7.5, 100 mM MgCl₂, 500 mM NaCl, 1 mg/ml BSA), 5 U *Sfi*I (Fermentas) and sufficient milliQ. The sample was incubated for 80 min at 50°C, and 20 min at 80°C to inactivate *Sfi*I. After cooling to RT, ligation was started by the addition of 1.5 µl 8 mM Na₂-ATP, pH 7, and 0.5 U T4 DNA ligase (Roche). The sample was incubated for 1 hr at 20°C and 20 min at 65°C to heat inactivate the T4 DNA ligase. Subsequently, 2 µl of the sample was transformed to 30 µl electrocompetent *L. lactis* NZ9000 (see below) and aliquots were plated on M17 plates (Terzaghi and Sandine, 1975) (Difco) supplemented with 0.5% glucose, 0.5 M sucrose, 5 µg/ml chloramphenicol. Parafilm-sealed plates were incubated at 30°C until colonies appeared (-18 hrs).
**Electrotransformation of *L.lactis.*** Preparation of electrocompetent *L. lactis* NZ9000 was essentially done as described (Holo and Nes, 1989; Wells et al, 1993), but with some critical modifications. Briefly, cells were grown in M17 supplemented with 0.5% glucose, 0.5 M sucrose and 2% glycine at 30°C to OD₆₀₀ = ~0.5. Cells were harvested by centrifugation at 5000 x g for 15 min at 4°C. Following washes with 1 volume ice-cold solution A (0.5 M sucrose and 10% glycerol, prepared in milliQ), 0.5 volume solution A supplemented with 50 mM Na-EDTA, pH 7.5, and 0.25 volume solution A, cells were resuspended in 0.01 volume solution A. Aliquots of 40 µl were flash-frozen in liquid nitrogen and stored at -80°C until use. For electroporation, cells were thawed on ice, combined with plasmid DNA, and transferred to an ice-cooled electroporation cuvet (2 mm gap). Cells were exposed to a single electrical pulse with a field strength of 2 kV, 25 µF capacitance and 200Ω resistance. Immediately following discharge, cells were mixed with 1 ml ice-cold M17 supplemented with 0.5% glucose, 0.5 M sucrose, 20 mM MgCl₂ and 2 mM CaCl₂, and left on ice for 10 min. Subsequently, cells were incubated at 30°C for 2 hrs and aliquots were plated on M17 agar supplemented with 0.5% glucose, 0.5 M sucrose and 5 µg / ml chloramphenicol. was added. Plates were sealed and incubated overnight at 30°C.

**Table 1. Analysis of the distribution of SfiI sites over pro- and eukaryotic gene transcripts. For the pro- and eukaryotic datapoints, 1.484.533 and 745.558 gene transcripts were analyzed, respectively.**

| *Sfi*I sites in transcript | Percentage of transcripts | |
|---|---|---|
| | prokaryotes | eukaryotes |
| 0 | 92.18 | 92.06 |
| 1 | 6.42 | 6.49 |
| 2 | 1.09 | 1.04 |
| 3 | 0.22 | 0.23 |
| 4 | 0.06 | 0.08 |
| 5 | 0.02 | 0.03 |
| 6 | 0.01 | 0.02 |
| 7 | 0.00 | 0.01 |
| 8 | 0.00 | 0.01 |
| 9 | 0.00 | 0.01 |
| >9 | 0.00 | 0.02 |

### Detailed overview of the LIC process.

**nLIC cassette:** A construct is made that contains an N-terminal 10 His-tag, followed by a TEV protease cleavage site and Your Favorite Protein (YFP). Using the 3'→5' exonuclease activity of T4 DNA polymerase and dedicated tail-sequences, long defined overhangs are generated. These overhangs have a sufficiently high annealing temperature that mere mixing of complementary overhangs suffices in generating stable DNA sequences, ready for cell transformation.
The vector holds the nLIC cassette which contains a *SwaI* site. After *SwaI* digestion, the vector is treated with T4 DNA polymerase in the presence of **dCTP**, in order to generate the *nLIC-ready* overhangs (illustrated below). 
1. nLIC cassette
N  Met His His His His His His His His His His Gly Glu Asn Leu Tyr
5′ ATG CAT CAT CAC CAT CAT CAC CAT CAC CAT CAT GGT GAG AAT TTA TAT TTAAATCCCACCCTCCCAG
3′ TAC GTA GTA GTG GTA GTA GTG GTA GTG GTA GTA CCA CTC TTA AAT ATA AATTTAGGGTGGGAGGGTC

2. After digestion with SwaI
N  Met His His His His His His His His His His Gly Glu Asn Leu Tyr
5′ ATG CAT CAT CAC CAT CAT CAC CAT CAC CAT CAT GGT GAG AAT TTA TAT TT  AAATCCCACCCTCCCAG
3′ TAC GTA GTA GTG GTA GTA GTG GTA GTG GTA GTA CCA CTC TTA AAT ATA AA  TTTAGGGTGGGAGGGTC

3. After treatment with T4 DNA polymerase + dCTP
N  Met His His His His His His His His His His Gly Glu Asn Leu Tyr
5′ ATG CAT CAT CAC CAT CAT CAC CAT CAC CAT C                    AAATCCCACCCTCCCAG
3′ TAC GTA GTA GTG GTA GTA GTG GTA GTG GTA GTA CCA CTC TTA AAT ATA AA                    C
 The insert is PCRed using primers with dedicated nLIC tails. After removal of primers and nucleotides, the insert is treated with T4 DNA polymerase in the presence of **dGTP,** in order to generate the nLIC-ready overhangs (illustrated below). 
1. PCR
N  is Gly Glu Asn Leu Tyr Phe Gln Gly Met      X   X
5′ AT GGT GAG AAT TTA TAT TTT CAA GGT ATG YFP TAA TGA AAA TCC CAC CCT CCC A
3′ TA CCA CTC TTA AAT ATA AAA GTT CCA TAC YFP ATT ACT TTT AGG GTG GGA GGG T

2. After treatment with T4 DNA polymerase + dGTP
N  is Gly Glu Asn Leu Tyr Phe Gln Gly      X   X
5′ AT GGT GAG AAT TTA TAT TTT CAA GGT YFP TAA TG
3′                            GTT CCA YFP ATT ACT TTT AGG GTG GGA GGG T
 Subsequently, the nLIC-ready vector and insert are mixed. The defined overhangs anneal to stable structures with a Tₘ of approximately 44°C and 58°C for the 5' and 3' end of the gene, respectively. Small, 1 basepair gaps remain, which will be filled *in vivo.* 
a. 5′ end, before annealing
N  Met His His His His His His His His His H                    is Gly Glu Asn Leu Tyr Phe Gln Gly
5′ ATG CAT CAT CAC CAT CAT CAC CAT CAC CAT C                    AT GGT GAG AAT TTA TAT TTT CAA GGT YFP
3′ TAC GTA GTA GTG GTA GTA GTG GTA GTG GTA GTA CCA CTC TTA AAT ATA AA.                    GTT CCA YFP

b. 5′ end, after annealing
N  Met His His His His His His His His His His Gly Glu Asn Leu Tyr Phe Gln Gly
5′ ATG CAT CAT CAC CAT CAT CAC CAT CAC CAT CAT GGT GAG AAT TTA TAT TTT CAA GGT YFP
3′ TAC GTA GTA GTG GTA GTA GTG GTA GTG GTA GTA CCA CTC TTA AAT ATA AA. GTT CCA YFP

1. 3′ end, before annealing
N  Met     X   X
5′ ATG YFP TAA TG                    AAA TCC CAC CCT CCC AG
3′ TAC YFP ATT ACT TTT AGG GTG GGA GGG T                    C

2. 3′ end, after annealing
N  Met     X   X
5′ ATG YFP TAA TG. AAA TCC CAC CCT CCC AG
3′ TAC YFP ATT ACT TTT AGG GTG GGA GGG TC
 **cLIC cassette:** A construct is made that contains a relatively small N-terminal modification (MGGGFA), Your Favorite Protein (YFP), and a C-terminal TEV protease cleavage site followed by a 10 His-tag. Using the 3→5' exonuclease activity of T4 DNA polymerase and dedicated tail-sequences, long defined overhangs are generated. These overhangs have a sufficiently high annealing temperature that mere mixing of complementary overhangs suffices in generating stable DNA sequences,ready for cell transformation.
The vector holds the cLIC cassette which contains a *SwaI* site. After *SwaI* digestion, the vector is treated with T4 DNA polymerase in the presence of **dCTP**, in order to generate the *nLIC-ready* overhangs (illustrated below). 
1. cLIC cassette
M G G G F N L Y F Q G H H H H H H H H H H X
5′ C ATG GGT GGT GGA TTT A AAT TTA TAC TTC CAA GGT CAT CAT CAC CAT CAT CAC CAT CAC CAT CAT TAA
3′ G TAC CCA CCA CCT AAA T TTA AAT ATG AAG GTT CCA GTA GTA GTG GTA GTA GTG GTA GTG GTA GTA ATT

2. After digestion with SwaI
M G G G F N L Y F Q G H H H H H H H H H H X
5′ C ATG GGT GGT GGA TTT A AAT TTA TAC TTC CAA GGT CAT CAT CAC CAT CAT CAC CAT CAC CAT CAT TAA
3′ G TAC CCA CCA CCT AAA T TTA AAT ATG AAG GTT CCA GTA GTA GTG GTA GTA GTG GTA GTG GTA GTA ATT

3. After treatment with T4 DNA polymerase + dCTP
M G G G F N L Y F Q G H H H H H H H H H H X
5′ C A AAT TTA TAC TTC CAA GGT CAT CAT CAC CAT CAT CAC CAT CAC CAT CAT TAA
3′ G TAC CCA CCA CCT AAA CCA GTA GTA GTG GTA GTA GTG GTA GTG GTA GTA ATT
 The insert is PCRed using primers with dedicated cLIC tails. After removal of primers and nucleotides, the insert is treated with T4 DNA polymerase in the presence of **dGTP**, in order to generate the cLIC-ready overhangs (illustrated below). 
1. PCR
M G G G F A E N L Y F Q
5' ATG GGT GGT GGA TTT GCT YFP GAA AAT TTA TAC TTC CAA
3' TAC CCA CCA CCT AAA CGA YFP CTT TTA AAT ATG AAG GTT

2. After treatment with T4 DNA polymerase + dGTP
M G G G F A E N L Y F Q
5' ATG GGT GGT GGA TTT GCT YFP G
3' GA YFP CTT TTA AAT ATG AAG GTT
 Subsequently, the cLIC-ready vector and insert are mixed. The defined overhangs anneal to stable structures with a Tm of approximately 41°C and 31°C for the 5' and 3' end of the gene, respectively. Small, 1 basepair gaps remain, which will be filled *in vivo.*

a. 5' end, before annealing
M G G G F A
5' C ATG GGT GGT GGA TTT GCT YFP
3' G TAC CCA CCA CCT AAA GA YFP

b. 5' end, after annealing
M G G G F A
5' C ATG GGT GGT GGA TTT GCT YFP
3' G TAC CCA CCA CCT AAA .GA YFP

1. 3' end, before annealing
E N L Y F Q N L Y F Q G H H H H H H H H H H X
5' YFP G A AAT TTA TAC TTC CAA GGT CAT CAT CAC CAT CAT CAC CAT CAC CAT CAT TAA
3' YFP CTT TTA AAT ATG AAG GTT CCA GTA GTA GTG GTA GTA GTG GTA GTG GTA GTA ATT

2. 3' end, after annealing
E N L Y F Q G H H H H H H H H H H X
5' YFP G.A AAT TTA TAC TTC CAA GGT CAT CAT CAC CAT CAT CAC CAT CAC CAT CAT TAA
3' YFP CTT TTA AAT ATG AAG GTT CCA GTA GTA GTG GTA GTA GTG GTA GTG GTA GTA ATT

### Brief description of the drawings

**Figure 1****.** Outline of the LIC-VBEx strategy. (a) A scheme illustrating the bisection of the *L. lactis* expression vector pNZxLIC. The segment containing the *cat* and LIC sequence is placed on an *E. coli* backbone, yielding pRExLIC. This vector can subsequently be used for the LIC procedure (depicted in b). Derivatives of pRExLIC containing inserts are converted to pNZxLIC derivatives by the VBEx procedure. (c) The *Sfi*I sites flanking both segments of the plasmid vectors yield different overhangs. (d) Overexpression of membrane proteins in *L*. *lactis.* Left panels represent Coomassie stained protein gels, right panels immunoblots decorated with anti-His antibody. Minus and plus signs indicate samples uninduced and induced with nisin A. Filled and open arrows indicate His-tagged and non-tagged subunits/proteins, respectively. Marker bands are 170, 130, 100, 70, 55, 40, 35, 25, and 15 kDa. Shown are, from left to right: an ABC transporter and secondary riboflavin transporter from *L. lactis,* ABC transporters from *Staphylococcus aureus* and *Streptococcus pneumoniae,* and the integral membrane component of a TRAP transporter from *Haemophilus influenzae;* accession numbers are indicated below the panels.

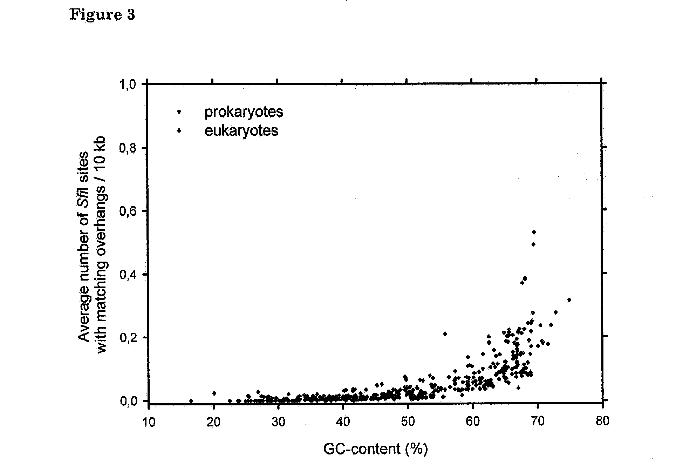

**Figure 2****.** Analysis of *Sfi*I characteristics. (a) The occurrence of *Sfi*I sites in 492 prokaryotic genomes and the concatinated gene transcripts of 30 eukaryotes as a function of the GC-content of the DNA. As 64 different overhangs can be generated after *Sfi*I digestion, the frequency of complementary *Sfi*I sites is even lower (Fig. 3). (b) The number of *Sfi*I overhangs not present in a genome or combined gene transcript as a function of the GC-content of the DNA (maximal 64). In rare cases where inserts would contain a *Sfi*I site interfering with the VBEx procedure, non-occuring overhangs could be used to flank both segments of the vector.
**Figure 3****.** Analysis of the occurrence of *Sfi*I sites yielding identical overhangs in genomes and combined transcripts as a function of the GC-content of the DNA. For each DNA, the datapoint of the most occurring *Sfi*I site of this type is shown.

### References

Aslanidis, C and P. J. de Jong, Nucleic Acids Res 18 (20), 6069 (1990).
Birnboim and Doly, Nucleic Acids Res. 1979 Nov 24;7(6):1513-1523
Bron PA, Benchimol MG, Lambert J, Palumbo E, Deghorain M, Delcour J, De Vos WM, Kleerebezem M, Hols P. Use of the alr gene as a food-grade selection marker in lactic acid bacteria. Appl Environ Microbiol. 2002 Nov;68(11):5663-70
de Jong, R. N. and M. A. Daniels, R. Kaptein et al., J Struct Funct Genomics (2007).
de Ruyter, P. G. and O. P. Kuipers, and W. M. de Vos, Appl Environ Microbiol 62 (10), 3662 (1996).
El Demerdash HA, Heller KJ, Geis A. Application of the shsp gene, encoding a small heat shock protein, as a food-grade selection marker for lactic acid bacteria. Appl Environ Microbiol. 2003 Aug;69(8):4408-12.
Guzman, L. M. and D. Belin, M. J. Carson et al., J Bacteriol 177 (14), 4121 (1995).
Holo, H and I. F. Nes, Appl Environ Microbiol 55 (12), 3119 (1989).
Li, M. Z. and S. J. Elledge, Nat Methods 4 (3), 251 (2007).
Liu, Q and M. Z. Li, and D. Leibham et al., Curr Biol 8 (24), 1300 (1998).
Surade, S. and M. Klein, P. C. Stolt-Bergner et al., Protein Sci 15 (9), 2178 (2006)
Terzaghi, B. E. and W. E. Sandine, Appl Microbiol 29 (6), 807 (1975).
Walhout, A. J. and G. F. Temple and M. A. Brasch et al., Methods Enzymol 328, 575 (2000).
Wells, J. M. and P. W. Wilson and R. W. Le Page, J Appl Bacteriol 74 (6), 629 (1993).

**Table 2**

| Available LIC vectors. | | | |
|---|---|---|---|
| **Vector name** | **Protein sequence** | **Protein sequence after TEV protease cleavage** | **Expression host** |
| pREnLIC | M-His₁₀-G-TEV site-protein | G-protein | *L. lactis* NZ9000 |
| pREcLIC | MGGGFA-protein-TEV site-His₁₀ | MGGGFA-protein-ENLYFQ | *L. lactis* NZ9000 |
| pREcLIC-GFP | MGGGFA-protein-TEV site-GFP-His₁₀ | MGGGFA-protein-ENLYFQ | *L. lactis* NZ9000 |
| pRE-USP45-nLIC | M-ssUSP45¹⁾-His₁₀-G-TEV site-protein | G-protein | *L. lactis* NZ9000 |
| pBADnLIC | M-His₁₀-G-TEV site-protein | G-protein | *E. coli* |
| pBADcLIC | MGGGFA-protein-TEV site-His₁₀ | MGGGFA-protein-ENLYFQ | *E. coli* |
| pBADcLIC-GFP | MGGGFA-protein-TEV site-GFP-His₁₀ | MGGGFA-protein-ENLYFQ | *E. coli* |
| pBAD-OmpA-nLIC | M-ssOmpA²⁾-His₁₀-G-TEV site-protein | G-protein | *E. coli* |

| | | | |
|---|---|---|---|
| ¹⁾ ssUSP45 indicates the signal sequence of the *L. lactis* USP45 protein. The pRE-USP45-nUC vector is used if the N-terminus of the membrane protein of interest is predicted to be on the outside of the cytoplasmic membrane, or to replace the signal sequence of the protein of interest with the ssUSP45. ²⁾ ssOmpA indicates the signal sequence of the *E. coli* OmpA protein. The pBAD-OmpA-nLIC vector is used if the N-terminus of the membrane protein of interest is predicted to be on the outside of the cytoplasmic membrane, or to replace the signal sequence of the protein of interest with the ssOmpA. | | | |

## Claims

1. A method for cloning a nucleic acid sequence of interest in a first kind of micro-organism, the method comprising:
- inserting said nucleic acid of interest into a nucleic acid vehicle, which vehicle comprises an origin of replication of a second kind of micro-organism;
- cloning said vehicle in a culture of said second kind of micro-organism;
- isolating cloned vehicle comprising said nucleic acid sequence of interest;
- substituting the vehicle's origin of replication of a second kind of micro-organism by an origin of replication of said first kind of micro-organism; and
- cloning the resulting vehicle in a culture of said first kind of micro-organism.

2. A method according to claim 1, wherein said resulting vehicle is essentially devoid of elements derived from said second kind of micro-organism.

3. A method according to claim 1 or 2, comprising:
- providing a first nucleic acid vehicle which comprises an origin of replication of said second kind of micro-organism;
- introducing a nucleic acid sequence of interest into said first vehicle;
- providing a second nucleic acid vehicle which comprises an origin of replication of said first kind of micro-organism; and
- substituting a part of said first vehicle, which part comprises said origin of replication of said second micro-organism but not said nucleic acid of interest, by a part of said second vehicle, said part comprising said origin of replication of said first microorganism.

4. A method according to claim 3, wherein said part of said first vehicle has different, non-palindromic overhangs that are not compatible with each other and that are compatible with the overhangs of said part of said second vehicle.

5. A method according to claim 3 or 4, wherein said parts are obtained by cleavage of said vehicles by *Sfi*I.

6. A method according to any one of claims 3-5, wherein said part of the first vehicle comprising an origin of replication of said second micro-organism comprises a selection marker, such as for instance an antibiotic resistance gene, and wherein said part of the second vehicle comprising an origin of replication of said first micro-organism comprises a selection marker, such as for instance an antibiotic resistance gene.

7. A method according to any one of claims 1-6, wherein said nucleic acid of interest is inserted into said first vehicle and cloned in a culture of said second kind of micro-organism using a method selected from the group consisting of a ligation independent cloning (LIC) procedure, Gateway, Univector Plasmid-fusion System (UPS) and LIC variants such as Enzyme-Free Cloning (EFC) and Sequence and Ligation Independent Cloning (SLIC).

8. A method according to any one of claims 3-7, wherein the process of inserting said nucleic acid of interest into said first vehicle and cloning it in a culture of said second kind of micro-organism is more efficient as compared to a process of inserting said nucleic acid of interest into said second vehicle and cloning it in a culture of said first kind of micro-organism.

9. A method according to any one of claims 1-8, wherein said second micro-organism is *Escherichia coli.*

10. A method according to any one of claims 1-9, wherein said first micro-organism is *Lactococcus lactis.*

11. A method according to any one of claims 1-10, further comprising allowing expression of said nucleic acid of interest by a culture of said first kind of micro-organism.

12. A method according to any one of claims 1-11, further comprising obtaining an expression product of said nucleic acid sequence of interest.

13. A method according to any one of claims 1-12, wherein said nucleic acid of interest encodes a multidomain protein and/or a membrane protein.

14. An expression product obtained by a method according to any one of claims 1-13.

15. A cloning assay wherein a nucleic acid of interest is cloned and expressed by a method according to any one of claims 1-13.

16. A kit of parts comprising:
- a first nucleic acid vehicle which comprises an origin of replication of a second kind of micro-organism; and
- a second nucleic acid vehicle which comprises an origin of replication of a first kind of micro-organism;
wherein each vehicle comprises at least two recombinase sites and/or restriction enzyme cleavage sites and wherein said recombinase sites and/or restriction enzyme cleavage sites are preferably not present within said origins of replication.

17. A kit of parts according to claim 16, wherein each vehicle comprises at least two restriction enzyme cleavage sites which, upon cleaving, yield two different, non-pahndromic overhangs that are not compatible with each other.

18. A kit of parts according to claim 16 or 17, wherein said first vehicle and said second vehicle comprise the same kind of restriction enzyme cleavage sites which, upon cleaving, yield two different, non-palindromic overhangs that are not compatible with each other.

19. A kit of parts according to any one of claims 16-18, wherein said restriction enzyme cleavage sites are *Sfi*I cleavage sites.

20. A kit of parts according to any one of claims 16-19, wherein said first vehicle comprises an *Escherichia coli* origin of replication.

21. A kit of parts according to any one of claims 16-20, wherein said second vehicle comprises a *Lactococcus lactis* origin of replication, a *Streptomyces* sp origin of replication and/or a *Sulfolobus solfataricus* origin of replication.

22. A kit of parts according to any one of claims 16-21, wherein said first vehicle comprises a selection marker, such as for instance an antibiotic resistance gene, and wherein said second vehicle comprises a selection marker, such as for instance an antibiotic resistance gene.

23. A *Lactococcus lactis* culture obtainable by a method according to any one of claims 1-13, wherein the amount of colony forming units per microgram nucleic acid of interest which was used in said method is at least 10⁵ CFU/µg DNA.

24. A recombinant micro-organism comprising a nucleic acid vector, which vector comprises:
- an origin of replication of said kind of micro-organism;
- a nucleic acid sequence of interest; and
- at least two restriction enzyme cleavage sites which, upon cleaving, yield two different, non-palindromic overhangs that are not compatible with each other and wherein said restriction enzyme cleavage sites are not present within said origins of replication.

25. A micro-organism according to claim 24, which is *Lactococcus lactis.*

26. A micro-organism according to claims 25, wherein said restriction enzyme cleavage sites are *Sfi*I cleavage sites.
